# EUROPEAN PATENT SPECIFICATION

(11) **EP 2 992 917 B1**
(45) Date of publication and mention of the grant of the patent: **08.08.2018**
(21) Application number: 14791910.4
(22) Date of filing: 25.03.2014
(51) Int. Cl.: A61M 5/32, A61M 5/158, A61M 5/34, B23K 13/01, B23K 26/20, B23K 26/324, B29C 65/14, B29C 65/34, B29C 65/36, B29K 23/00, B23K 103/00, B23K 103/04, B23K 103/10, B23K 103/14, B23K 103/18, B29L 31/00

(54) **OUTER CYLINDER WITH NEEDLE AND METHOD FOR PRODUCING SAME**
AUSSENZYLINDER MIT NADEL UND VERFAHREN ZUR HERSTELLUNG DAVON
CYLINDRE EXTÉRIEUR À AIGUILLE ET SON PROCÉDÉ DE PRODUCTION

(30) Priority: 02.05.2013 JP 2013096931
(43) Date of publication of application: 09.03.2016
(73) Proprietor: Terumo Kabushiki Kaisha, Tokyo 151-0072 (JP)
(72) Inventor: SUGIKI, Tsutomu, Nakakoma-gun Yamanashi 409-3853 (JP); GOTO, Kenta, Nakakoma-gun Yamanashi 409-3853 (JP)
(74) Representative: Casalonga
(86) International application number: PCT/JP2014/058202
(87) International publication number: WO 2014/178242

(56) References cited:
- WO-A1-2011/004728
- WO-A1-2012/034648
- WO-A1-2012/034648
- WO-A1-2012/043161
- WO-A1-2012/043161
- WO-A1-2012/043544
- JP-A- 2003 245 348
- JP-A- 2004 154 210
- JP-A- 2012 254 102
- JP-A- 2012 254 102
- US-A- 3 430 627
- US-A1- 2002 138 042

## Description

### TECHNICAL FIELD

The present invention relates to a needle-mounted outer cylinder in which a needle tube of a syringe is directly joined with a distal end of an outer cylinder of the syringe in advance and a method of producing the needle-mounted outer cylinder.

In particular, the invention relates to a needle-mounted outer cylinder according to the preamble of claim 1 and to a method for producing a needle-mounted outer cylinder according to the preamble of claim 4.

### BACKGROUND ART

When a medicine is injected subcutaneously or intradermally for disease prevention, disease diagnosis or disease treatment, administration of various vaccines is exemplified as the disease prevention. Administration of tuberculin is exemplified as the disease diagnosis. Administration of protein preparation and antibody drug formulation such as insulin, growth hormone, erythropoietin, granulocyte colony-stimulating factor is exemplified as the disease treatment. Because the dose of these medicines is mostly small, syringes having a small capacity (as the capacity, not more than several milliliters and more specifically, 1mL to 5mL) are normally used.

In a syringe having a small capacity, there is a case in which a needle-mounted outer cylinder in which a needle tube of a syringe is directly joined with a distal end of an outer cylinder of the syringe in advance is used. As methods of producing such needle-mounted outer cylinders, a method of joining the needle tube with the distal end of the outer cylinder with an adhesive agent or the like, a method of joining the needle tube with the distal portion of the outer cylinder by insert molding, and in addition, a method of heat-welding the needle tube to the outer cylinder (see patent document 1) are known. In a patent document 2, there is disclosed the needle-mounted outer cylinder having the barrel 1 tubularly formed and the needle 2 fixed to the barrel 1. The barrel 1 has the tubular needle fixing part 12 to which the needle 2 inserted thereinto is bonded at the distal part thereof. The needle fixing part 12 has the welding part 12a which is thermoplastic and heated to weld the needle 2 thereto. The welding part 12a is disposed at the position axially apart from the distal end of the needle fixing part 12.

WO 2012/034648 A1 discloses a syringe body/needle assembly wherein the needle is bonded to a needle holder via radiation applying heat to the needle holder material surrounding the needle.

### PRIOR ART DOCUMENT

### PATENT DOCUMENT

Patent document 1: Japanese Patent Application Laid-Open Publication Number 2004-154210
Patent document 2: Japanese Patent Application Laid-Open Publication Number 2012-254102

### SUMMARY OF THE INVENTION

### PROBLEMS TO BE SOLVED BY THE INVENTION

In the case where the insert molding is used as disclosed in the patent document 1, it is necessary to perform the work of disposing the needle tube having the blade surface formed thereon inside a die. There is a possibility that the blade surface may be damaged during this work. In the case where the joining member is used as disclosed in the patent document 2, it is necessary to perform the work of inserting the joining member into the distal portion of the outer cylinder and insert the needle tube into the joining member. In addition, in joining the needle tube with the joining member, it is necessary to perform the work of welding the needle tube with the joining member and heat-sealing the joining member with the outer cylinder. Therefore there is a possibility that the needle tube joining work may be complicated.

In the needle-mounted barrel of the patent document 2, the joining member is not used. The needle fixing part has the supporting portion which supports the needle radially with the needle fixing part in contact with the outer peripheral portion of the needle. The welding part is disposed at the position axially apart from the proximal end of the needle fixing part. The supporting portion is disposed at both sides of the welding part in the axial direction of the barrel. In the needle-mounted barrel of the patent document 2, it is necessary to perform the operation of inserting the needle into the small-diameter supporting portion which contacts the outer periphery of the needle. Insertion resistance is applied to the needle in the insertion operation. Thus the workability in the production process is low. In addition, in dependence on the dimension of inner diameters of portions other than the supporting portion, there is a possibility that the amount of the melted resin forming the supporting portion is insufficient for fixing the needle to the welding part.

Upon present inventors' earnest investigations, it is effective as a method of producing the needle-mounted outer cylinder of this type to apply energy to a metal needle tube from outside to allow the needle tube to generate heat and fusion-fix the needle tube to the outer cylinder by utilizing the generated heat. But the present inventors have found that in the case where the needle tube is fusion-fixed to the outer cylinder by using this method, foaming occurs at a melted portion and thus there is a possibility that foaming-caused defective fixing occurs.

The present invention has been made in view of the above-described problems. Thus it is an object of the present invention to provide a needle-mounted outer cylinder which allows a needle tube insertion work to be performed easily by setting into a needle insertion hole formed at a distal part of the outer cylinder larger than an outer diameter of the needle tube and which hardly generates foaming at a melt-bond portion and foaming-caused defective fixing of the needle tube to the outer cylinder, and a method of producing the needle-mounted outer cylinder.

### MEANS FOR SOLVING THE PROBLEMS

The above-described objects are achieved by a needle-mounted outer cylinder according to claim 1 and a method for producing a needle-mounted outer cylinder according to claim 4. The dependent claims relate to advantageous embodiments.

### BRIEF DESCRIPTION OF THE DRAWINGS

Fig. 1 is a front view of a needle-mounted outer cylinder of the present invention.
Fig. 2 is a plan view of the needle-mounted outer cylinder of the present invention.
Fig. 3 is a sectional view of the needle-mounted outer cylinder shown in Fig. 2 taken along an A-A line.
Fig. 4 is a main part-depicted vertical sectional view of the needle-mounted outer cylinder of the present invention.
Fig. 5 is a vertical sectional view of the outer cylinder.
Fig. 6 is an exploded perspective view of the needle-mounted outer cylinder of the present invention.
Fig. 7 is a front view of the needle-mounted outer cylinder of the present invention on which a cap is mounted.
Fig. 8 is a sectional view of the needle-mounted outer cylinder shown in Fig. 7 taken along a B-B line.
Fig. 9 is an explanatory view for explaining a needle fixing process to be performed in a method of producing the needle-mounted outer cylinder of the present invention.
Fig. 10 is an explanatory view for explaining the needle fixing process in the method of producing the needle-mounted outer cylinder of the present invention.
Fig. 11 is an explanatory view for explaining a needle fixing process to be performed in a method of producing a needle-mounted outer cylinder of another embodiment of the present invention.
Fig. 12 is an explanatory view for explaining a welding process of welding a needle tube to an outer cylinder of another embodiment in the method of producing the needle-mounted outer cylinder of the present invention.
Fig. 13 is a graph showing an example of a mode of imparting energy to a needle tube in the method of producing the needle-mounted outer cylinder of the present invention.
Fig. 14 is a graph showing a heat-generated state of the needle tube when energy is imparted thereto.

### MODE FOR CARRYING OUT THE INVENTION

The needle-mounted outer cylinder of the present invention is described below by using the embodiments shown in the drawings.

A needle-mounted outer cylinder 1 of the present invention is composed of a metal needle tube 3 having a lumen penetrating therethrough from its distal end to proximal end and an outer cylinder 2, made of thermoplastic resin, which has a distal part 22 having a needle insertion hole 41 into which a proximal portion of the needle tube 3 is inserted. The proximal portion 31 of the needle tube 3 is bonded to the outer cylinder with a melt-bond portion 45 formed inside the needle insertion hole 41 by melting an inner surface of the needle insertion hole 41 of the outer cylinder 2. The needle insertion hole 41 has an inner diameter a little larger than an outer diameter of the proximal portion 31 of the needle tube 3. The melt-bond portion 45 is positioned at a side distal by a predetermined length from a proximal end of the needle insertion hole 41 and is formed by partly melting the inner surface of the needle insertion hole 41 and by filling a gap between an outer surface of the proximal portion 31 of the needle tube 3 and the inner surface of the needle insertion hole with a portion of the inner surface of the needle insertion hole which has melted and flowed down toward a proximal side of the needle insertion hole. The distal part 22 of the outer cylinder 2 does not substantially have bubbles to be formed when the melt-bond portion 45 is formed.

The needle-mounted outer cylinder of the present invention is used to inject a medicine into a living body by puncturing a needle tip into the surface of skin.

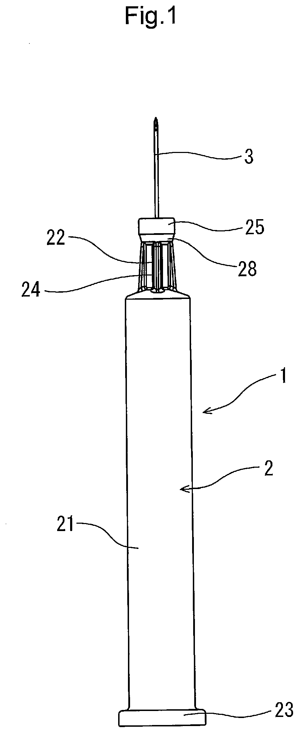

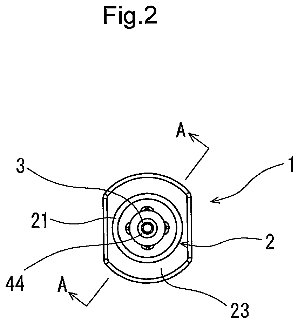

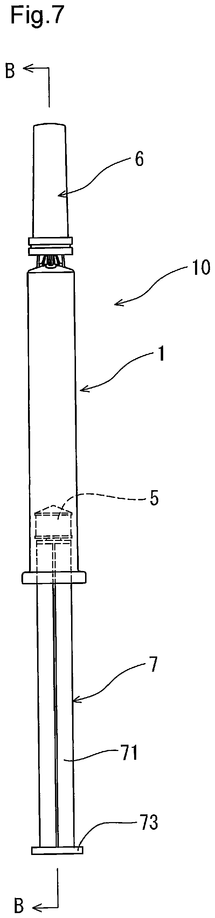

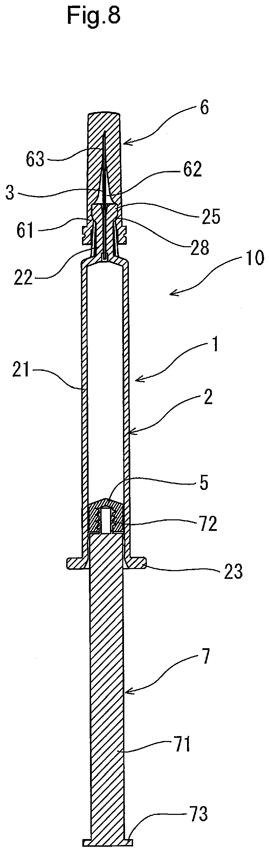

As shown in Figs. 1 through 4, the needle-mounted outer cylinder 1 of this embodiment has the outer cylinder 2 and the needle tube 3 fixed to the inside of the distal part of the outer cylinder 2. As shown in Figs. 7 and 8, a cap 6 is mounted on the needle-mounted outer cylinder 1.

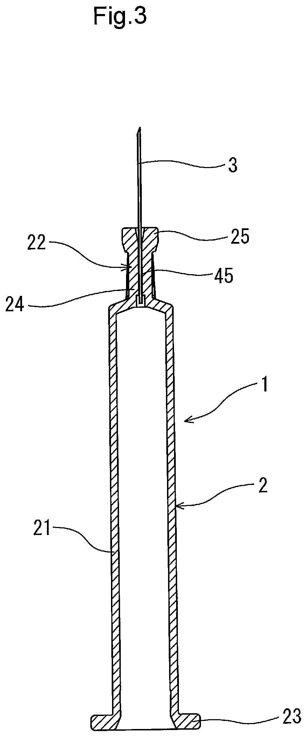

As shown in Figs. 1 and 3, needle tubes of 27 to 30G (outer diameter: 0.41 to 0.31mm) conforming to the ISO standard for a medical needle tube (ISO 9626: 1991/Amd. 1:2001(E)) are used as the needle tube 3.

The needle tube 3 has the lumen penetrating therethrough from its distal end to proximal end. The needle tube 3 has a needle tip 32 to be punctured into the living body at its distal end. The needle tip 32 having a blade surface is formed at an acute angle. A distal end portion of the needle tube 3 including the needle tip 32 projects from the distal end of the distal part 22 of the outer cylinder 2. A proximal end portion of the needle tube 3 is accommodated inside the needle insertion hole 41 of the outer cylinder 2. In this embodiment, the proximal end of the needle tube 3 penetrates through the needle insertion hole and reaches the inside of the outer cylinder 2.

The proximal side portion of the needle tube 3 is positioned inside the needle insertion hole 41. It is preferable that a surface of a portion of the needle tube 3 to be fixed to the outer cylinder is formed as a rough surface by blast treatment. By so doing, when the needle tube 3 is bonded to the outer cylinder 2 by means of welding, softened resin enters into concaves and convexes of the rough surface of the needle tube 3. Thereby it is possible to join the needle tube 3 with the outer cylinder 2 at a high joint strength. Further, because the softened resin enters into the concaves and the convexes of the rough surface of the needle tube 3, the needle-mounted outer cylinder of the present invention has an improved liquid-tightness. Thus the needle-mounted outer cylinder can be preferably used for a syringe.

As materials for the metal needle tube 3, stainless steel is preferable. But the materials therefor are not limited to stainless steel. It is possible to use aluminum, aluminum alloys, titanium, titanium alloys, and other metals. As the needle tube 3, it is possible to use not only a straight needle conforming to the ISO standard, but also a needle having large or small outer and inner diameters. More specifically, in addition to needles of 31G or 32G, it is possible to use a tapered needle in which least one portion is tapered. The needle tube 3 may have a polygonal configuration in addition to a circular configuration in its sectional configuration. A coating agent consisting of silicone resin or fluororesin may be applied to the surface of the needle tip 32 of the needle tube 3.

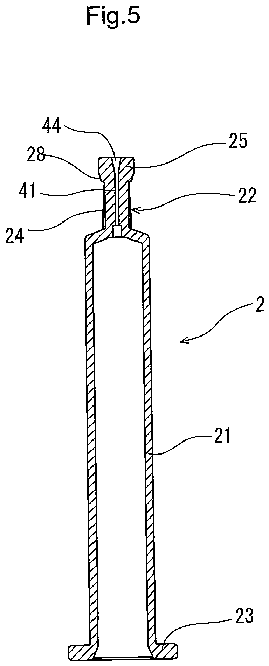

The outer cylinder 2 is described below. As shown in Fig. 5, the outer cylinder 2 has a main body part 21 in which a medicine is filled and a distal part 22 having the needle insertion hole 41. The main body part 21 has an internal accommodation portion and is formed substantially cylindrically. The main body part 21 has a flange 23 formed at its rear-end side in its axial direction.

The distal part 22 has a distal bulged portion 25 and a tubular portion 24 connecting the distal bulged portion 25 and the distal end of the main body part 21 to each other. The distal part 22 has the needle insertion hole 41 extending into the main body part 21 from the distal end thereof. It is favorable to form the inner diameter of the needle insertion hole 41 larger than the outer diameter of the needle tube 3 by 0.02mm to 0.14mm and more favorable by 0.02mm to 0.09mm. In the case where the needle tube 3 of 27G to 30G is used, by setting the inner diameter of the needle insertion hole 41 to 0.43 to 0.45mm, the gap between the needle insertion hole 41 and the needle tube 3 can be set as described above. When any needle tube 3 whose outer diameter at the position of a melt-bond portion corresponds to 27 to 30G is used, it is possible to secure the joint strength of the needle tube 3 and prevent the needle tube 3 joined with the outer cylinder from tilting more than a predetermined angle after the needle tube is joined with the outer cylinder by setting the inner diameter of the needle insertion hole 41 to the above-described range. It is preferable that the thickness between the outer surface of the distal part 22 of the outer cylinder 2 and the needle insertion hole 41 is 1.2 to 1.5mm. By setting the above-described thickness to the above-described range, energy can be efficiently imparted to the needle tube, and the outer surface of the distal part thereof does not easily melt. Thereby after the needle tube is joined with the outer cylinder, the outer cylinder has a high strength. Further it is possible to secure the joint strength of the needle tube 3 and prevent the needle tube 3 joined with the outer cylinder from tilting more than the predetermined angle.

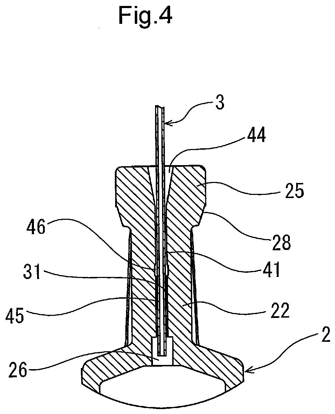

As shown in Fig. 4, a distal end portion of the needle insertion hole 41 is formed as a distal diameter enlarged portion 44 having a diameter larger than those of other portions thereof. This construction of the needle insertion hole allows the proximal portion 31 of the needle tube to be easily inserted into the needle insertion hole 41. The proximal end of the needle insertion hole 41 is formed as a proximal diameter enlarged portion 26. The proximal end of the needle tube 3 enters into the proximal diameter enlarged portion 26 and does not reach the inside of the main body part 21.

In this embodiment, an example in which the main body part 21 is formed approximately cylindrically is described. But the main body part 21 may be hollow square columnar or hollow hexagonal columnar.

As materials to form the outer cylinder 2, various resins such as polyvinyl chloride, polyethylene, polypropylene, cyclic polyolefin, polystyrene, poly-(methylpentene-1), polycarbonate, acrylic resin, acrylonitrile-butadiene-styrene copolymer, polyester such as polyethylene terephthalate, butadiene-styrene copolymer, and polyamide (for example, nylon 6, nylon 66, nylon 610, and nylon 12) are listed. Of these resins, it is preferable to use the polypropylene, the cyclic polyolefin, and the poly-(methylpentene-1). It is preferable that materials to form a joining member 4 and the outer cylinder 2 are substantially transparent to secure visibility of the inside thereof.

As the cyclic polyolefin, it is possible to preferably use cyclic polyolefin formed from components obtained by ring-opening metathesis polymerization of norbornene or norbornene derivatives, cyclic olefin polymer (COP) which is a hydrogenated product of the cyclic polyolefin, and cyclic olefin copolymer (COC) formed by copolymerization of norbornene and ethylene. In the cyclic polyolefin, in consideration of injection moldability in molding a material into an outer cylinder and the formability of the melt-bond portion in joining the needle tube with the outer cylinder, it is preferable to use cyclic polyolefins having the following MFR (melt flow rate) and glass transition temperature. That is, the MFR is favorably in the range of 10 to 40g/10 minutes and more favorably in the range of 10 to 35g/10 minutes. When the MFR is not less than 10g/10 minutes, the flowability of the cyclic polyolefin does not become too low and the resin is capable of sufficiently entering into the concave-convex surface. Thus the cyclic polyolefin allows the needle tube to be joined with the outer cylinder at a high joining strength. In the case where resin having the MFR lower than 10g/minute is used, it is possible to obtain a desired joining strength by pressurizing the outer cylinder to press the needle tube within a range not departing from the gist of the present invention, although it depends on the degree of the MFR having a low MFR. In the case where the MFR is not more than 40g/minute, the flowability of the resin does not become too high, and melted resin stays at a position where the melt-bond portion is to be formed. Therefore it is possible to form a satisfactory melt-bond portion. The glass transition temperature is favorably in the range of 100 to 160 degrees C. Considering a case where the needle-mounted outer cylinder of the present invention is sterilized by using a high pressure steam in an overkill condition, the glass transition temperature is more favorably in the range of 125 to 160 degrees C.

As the COP to be used in the present invention, ZEONEX (registered trademark, produced by Nippon Zeon Co., Ltd.) and Daikyo Resin CZ (produced by Daikyo Seiko, Ltd.) are exemplified. As the COC, APEL (registered trademark, produced by Mitsui Chemicals Co., Ltd.), TOPAS (registered trademark, produced by Topas Advanced Polymers Gesellschaft mit beschrankter Haftung) are exemplified.

As shown in Figs. 1 through 4, the needle tube 3 is welded to the outer cylinder 2 by using a production method described later to construct the needle-mounted outer cylinder 1. As shown in Fig. 4, the needle tube 3 is bonded to the outer cylinder 2 with the melt-bond portion 45 formed at a position disposed at a side proximal by a predetermined length from the distal end of the needle insertion hole 41 of the distal part 22 of the outer cylinder 2 and at a position disposed at a side distal from the proximal end of the needle insertion hole 41 by a predetermined length. More specifically, the proximal portion 31 of the needle tube 3 is bonded to the outer cylinder 2 at the position disposed at the side distal from the proximal end of the needle insertion hole 41 by the predetermined length. The proximal end of the melt-bond portion 45 is formed at a position disposed at a side distal by a short distance from the distal end of the proximal diameter enlarged portion 26 of the needle insertion hole 41. The distal end of the melt-bond portion 45 is formed at a position disposed at a side proximal from the distal diameter enlarged portion by a predetermined length. In this embodiment, the needle insertion hole has a diameter enlarged portion (diameter enlarged portion formed of melted surface) 46 formed of a melted surface extended to a side distal from the distal end of the melt-bond portion 45 by a short distance. The distal part 22 of the outer cylinder 2 including the melt-bond portion 45 does not substantially have bubbles to be generated owing to the formation of the melt-bond portion 45. To prevent the bubbles from being formed, it is preferable to form the melt-bond portion 45 by heating the needle tube to not less than the melting temperature of thermoplastic resin and less than the thermal decomposition temperature thereof and thereafter solidifying the thermoplastic resin (without interposing other heating processes). The length of the melt-bond portion 45 is set to favorably 2.1 to 5.1mm.

A syringe 10 on which the needle-mounted outer cylinder 1 of the present invention is mounted is described below.

As shown in Fig. 7, the syringe 10 has the needle-mounted outer cylinder 2, the cap 6 mounted on the distal part (needle part) of the outer cylinder 2, a gasket 5 accommodated inside the outer cylinder 2 and being slidable inside the outer cylinder, and a plunger 7 mounted on the gasket 5. The plunger 7 has a main body part 71, a gasket mounting part 72 formed at a distal end of the main body part 71, and a pressing part 73 formed at a proximal portion of the plunger. The gasket has a plunger mounting part which receives the gasket mounting part 72 of the plunger 6 and engages the gasket mounting part 72.

The cap 6 is formed in a cylindrical configuration, open at a proximal portion 61 thereof in its axial direction, and closed at a distal end thereof in its axial direction. The cap 6 is formed of an elastic member such as rubber and elastomer. The cap 6 is mounted on the distal part 22 of the outer cylinder 2 in such a way as to cover the needle tip 32 of the needle tube 3 and the distal part 22 of the outer cylinder 2. As shown in Fig. 7, the needle tube 3 and the distal part 22 are inserted into a lumen portion 62 of the cap 6.

The inner diameter of the lumen portion 62 of the cap 6 is formed almost equally to the outer diameter of the distal bulged portion 25 of the distal part 22 or a little smaller than the outer diameter of the distal bulged portion 25. Therefore when the cap 6 is mounted on the distal part 22, the outer peripheral surface of the distal bulged portion 25 closely contacts the inner peripheral surface of the cap 6. Thereby a space covering the needle tube 3 projected from the outer cylinder 2 is closed with the distal bulged portion 25 and the inner peripheral surface of the cap 6. This construction is capable of preventing bacteria from sticking to the needle tip 32. At the same time, the needle tip holding portion 63 holds the needle tip 32.

An annular rib 61 formed on the inner peripheral surface of the cap 6 tightens a constricted portion disposed at the boundary between the distal bulged portion 25 of the distal part 22 and a tapered fitting portion 28 thereof by an elastic force of the annular rib 61. Owing to the engagement between the inner peripheral surface of the cap 6 and the constricted portion of the distal part 22, it is possible to prevent the cap 6 from being removed from the distal part 22 during delivery.

The method of producing the needle-mounted outer cylinder 1 is described below.

The production method of the present invention is to produce the needle-mounted outer cylinder composed of the metal needle tube 3 having the lumen penetrating therethrough from its distal end to proximal end and the outer cylinder 2, made of thermoplastic resin, which has the distal part 22 having the needle insertion hole 41 into which the proximal portion 31 of the needle tube 3 is inserted. The proximal portion 31 of the needle tube 3 is bonded to the outer cylinder with the melt-bond portion 45 formed inside the needle insertion hole 41 by melting the inner surface of the needle insertion hole 41 of the outer cylinder 2. Specifically, it is a method for producing the needle-mounted outer cylinder 1 described above.

The method of producing the needle-mounted outer cylinder of the present invention consists of execution of a melt-bond process of melting and bonding the proximal portion 31 of the needle tube 3 to the outer cylinder in a state in which the needle tube 3 is inserted into the needle insertion hole 41 of the outer cylinder 2 and in a state in which the outer cylinder 2 is erect. The melt-bond process is performed by imparting energy to the needle tube 3 at a portion thereof positioned at a side distal from the proximal end of the needle insertion hole 41 and from the proximal end of the needle tube to allow the needle tube 3 to generate heat so that resin which is positioned at a heat generation portion (energy imparting region) and forms the inner surface of the needle insertion hole 41 is melted, and thereafter by flowing the melted resin downward inside the needle insertion hole 41 to thereby fill the gap between the outer surface of the proximal portion 31 of the needle tube 3 and the needle insertion hole 41.

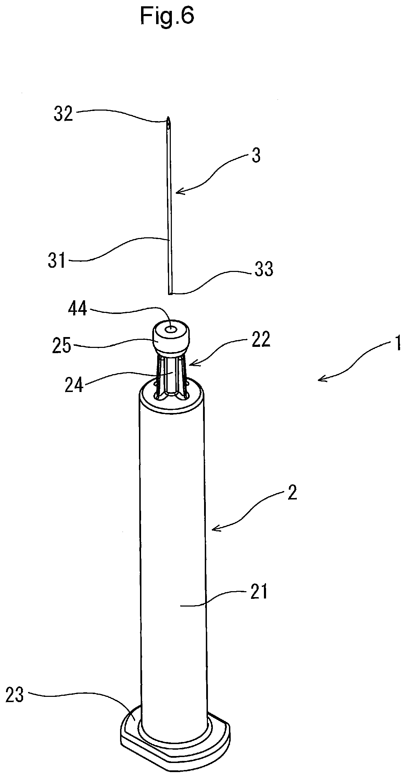

To produce the needle-mounted outer cylinder 1 of the present invention, as shown in Fig. 6, the metal needle tube 3 and the outer cylinder 2 made of thermoplastic resin are prepared. The needle tube 3 is formed as a desired tubular body by press working of a flat metal or swaging processing of a hollow pipe. The outer cylinder 2 is formed by injection-molding the thermoplastic resin.

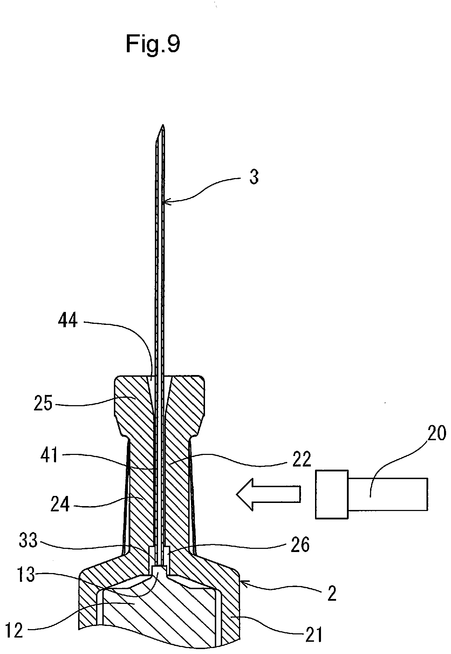

Thereafter the process of joining the needle tube 3 with the outer cylinder 2 is performed. In this process, initially the needle tube 3 is inserted into the distal diameter enlarged portion 44 of the needle insertion hole 41 of the outer cylinder 2 from the proximal side of the needle tube. As shown in Fig. 9, in this embodiment, the needle tube 3 is placed in position with respect to the outer cylinder 2 by using a needle supporting member 12. The needle supporting member 12 is disposed inside the internal accommodation portion of the main body part 21 and has a supporting projection 13 which supports the proximal end 33 of the needle tube 3 at the upper end thereof. The needle supporting member 12 is so disposed that the supporting projection 13 thereof is positioned inside the proximal diameter enlarged portion 26 of the distal part 22. The proximal end of the needle tube 3 supported by the supporting projection 13 is positioned inside the proximal diameter enlarged portion 26 of the needle insertion hole 41 and does not project into the internal accommodation portion of the main body part 21. Therefore it is possible to decrease the dead volume inside the outer cylinder 2, decrease the amount of the medicine remaining inside the outer cylinder 2, and prevent the distal end of the gasket from contacting the proximal end of the needle tube 3.

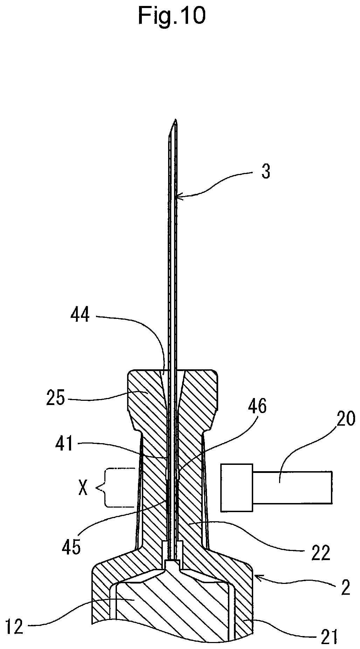

Thereafter the melt-bond process is performed. As shown in Fig. 10, in this embodiment, the melt-bond process is performed by using a semiconductor laser irradiation apparatus 20. The semiconductor laser irradiation apparatus 20 emits laser to a portion of the needle tube 3 corresponding to an energy imparting region X shown in Fig. 10. Thereby the portion of the needle tube 3 irradiated with the laser generates heat. Owing to the heat generated by the needle tube, the inner surface of the needle insertion hole 41 proximate to the outer surface of the heat generation portion is heated by radiation (heat transfer) and melts.

Because the outer cylinder 2 is erect, the melted resin drops by gravity. When the melted resin passes the energy imparting region (moves away from the heat generation portion of the needle tube), the melted resin hardens and fills the gap between the surface of the needle insertion hole and the needle tube to form the lower end portion of a welding portion. In this manner, the energy imparting operation finishes. The melt-bond process finishes when the resin hardens.

In the melt-bond process of the production method of the present invention, it is unnecessary to press a heating portion unlike the melt-bond process performed in the production method of the patent document 2. Because the heating portion is pressed, there is a possibility that deformation and distortion-caused crack may occur. On the other hand, in the present invention, because it is unnecessary to perform a pressing operation, neither pressing-caused deformation nor pressing-caused crack occurs. Thus it is preferable to carry out the melt-bond process in such a way that the outer surface of the distal part 22 of the outer cylinder 2 does not reach the melting temperature of the resin and in a state in which a stress loading is not applied to the distal part 22 from the outer surface thereof.

In the needle-mounted outer cylinder 1 formed by carrying out the production method of the present invention, as shown in Fig. 4, the diameter enlarged portion 46 is formed at a position inside the distal part of the outer cylinder corresponding to the distal portion of the energy imparting region X owing to the melting and flow-down of the resin.

It is preferable that the melt-bond process has a needle tube heating process of allowing the needle tube to generate heat in such a way that the thermoplastic resin heating temperature at the heat generation portion (energy imparting region) X becomes not less than the melting temperature of the thermoplastic resin and less than the thermal decomposition temperature thereof. By performing this process, it is possible to prevent a bubble-holding portion from being formed inside the thickness of the distal part 22 of the outer cylinder 2 and outside the melt-bond portion 45.

It is possible to perform the melt-bond process by heating the entire needle tube in such a way that the thermoplastic resin heating temperature becomes not less than the melting temperature thereof and less than the thermal decomposition temperature thereof. To securely fix the needle tube to the outer cylinder in this method, a certain period of time is necessary to impart energy to the needle tube. Thus the working period of time is long. In addition, there is a possibility that the outer surface side of the energy imparting region may be heated and softened beyond the inner surface of the needle insertion hole of the outer cylinder.

It is preferable for the melt-bond process to carry out an initial heating process of allowing the needle tube to generate heat in such a way that the thermoplastic resin heating temperature at the heat generation portion (energy imparting region) X exceeds the thermal decomposition temperature of the thermoplastic resin and a final heating process of allowing the needle tube to generate heat in such a way that the thermoplastic resin heating temperature at the heat generation portion becomes not less than the melting temperature of the thermoplastic resin and less than the thermal decomposition temperature thereof, after the execution of the initial heating process finishes.

By doing so, it is possible to fix the needle tube to the outer cylinder securely and in a short period of time and prevent the bubble-holding portion from being formed inside the thickness of the distal part 22 of the outer cylinder 2 and outside the melt-bond portion 45.

In the initial heating process (strong heating process), it is preferable to allow the needle tube to generate heat in such a way that the temperature of the resin at the heating portion becomes not less than the thermal decomposition temperature of the resin and not more than +25% thereof. In the initial heating process (strong heating process), it is particularly preferable to allow the needle tube to generate heat in such a way that the temperature of the resin at the heating portion becomes not less than the thermal decomposition temperature of the resin by not less than +5% and not more than +20% thereof. It is also preferable to perform the initial heating process (strong heating process) in such a way that the outer surface of the distal part of the outer cylinder does not reach the melting temperature of the resin. More specifically, by arranging the intensity of energy to be imparted to the needle tube and an energy imparting period of time, it is possible to accomplish the execution of the initial heating process. It is particularly preferable to perform the initial heating process by imparting a high energy to the needle tube in a short period of time. A portion of the needle insertion hole confronting (proximate to) the heating portion of the needle tube is strongly heated. Thus the resin at this portion securely melts and flows downward.

More specifically, in the initial heating process (strong heating process), it is preferable to set the output of the semiconductor laser irradiation apparatus 20, the laser focus diameter, the length of the energy imparting region (heating region), and the laser irradiation period of time to 10 to 20W, ϕ2 to 5mm, +0.1mm (2.1 to 5.1mm) of the laser focus diameter, and 0.5 to 1.5 seconds respectively.

In the final heating process, it is preferable to heat the needle tube in such a way that the temperature of the resin at the heating portion becomes not more than the thermal decomposition temperature of the thermoplastic resin and not less than the melting temperature thereof. It is preferable to carry out the final heating process in such a way that the temperature of the outer surface of the distal part of the outer tube does not reach the melting temperature of the resin. More specifically, by arranging the intensity of the energy to be imparted to the needle tube and the energy imparting period of time, it is possible to perform the execution of the final heating process. For example, it is preferable to carry out the final heating process by imparting energy lower than that used in the initial heating process to the needle tube for a predetermined period of time. In the final heating process, it is preferable to allow the needle tube to generate heat in such a way that the temperature of the resin at the heating portion becomes lower than the thermal decomposition temperature of the resin by 5% to 20%. In the final heating process, it is preferable to allow the needle tube to generate heat in such a way that the temperature of the resin at the heating portion becomes higher than the melting temperature of the resin by at least 60 degrees C.

In the final heating process, it is preferable to maintain a laser focus diameter and the length of an energy imparting region (heating region) in the initial heating process and set the output of the semiconductor laser irradiation apparatus 20 to 40% to 60% of the output in the initial heating process. More specifically, it is preferable to set the output of the semiconductor laser irradiation apparatus to 5 to 20W. It is also preferable to set an irradiation period of time of the laser to 0.5 to 2.5 seconds.

It is preferable to set the total of the period of time required to perform the initial heating process and that required to perform the final heating process to one to five seconds. An intermediate heating process may be performed at a temperature not more than the thermal decomposition temperature of the resin between the execution of the initial heating process and that of the final heating process. The intermediate heating process may be performed at a temperature not more than the melting temperature of the resin.

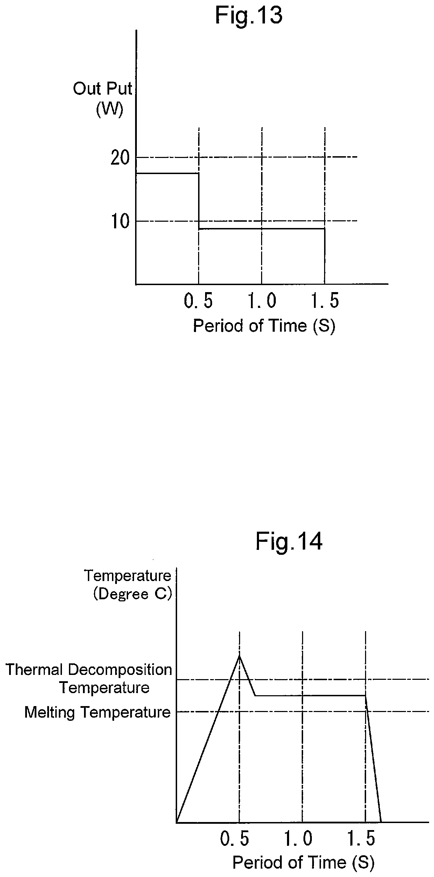

As the output imparting mode in which the semiconductor laser irradiation apparatus heats the needle tube, it is preferable to apply laser to the needle tube as shown in Fig. 13. In this example, initial heating is performed by applying the laser to the needle tube at an output of 20w for 0.5 seconds. Thereafter the laser is applied to the needle tube for one second by decreasing the output to 10w. As shown in Fig. 14, in imparting the energy to the needle tube as in the case of this example, after the needle tube is initially heated to quickly increase the temperature thereof to not less than the thermal decomposition temperature of the resin, the temperature of the needle tube is maintained at a temperature less than the thermal decomposition temperature of the resin and not less than the melting temperature thereof for a predetermined period of time. Thereafter the temperature of the needle tube returns to a normal temperature.

In the above description, the initial heating process in a melting/heating process is performed at a first output level (high level), while the final heating process is performed at a second output level (low level) lower than the first output level. But alternatively, the final heating process may be performed by gradually lowering the first output level set in the initial heating process to allow the needle tube to generate heat.

By adjusting the position at which the melt-bond portion 45 is to be formed, the flexibility (deflection) of the needle tube can be controlled. For example to prevent the needle tube from kinking (bending) by allowing the needle tube to easily bend, it is preferable to form the melt-bond portion 45 at a position proximate to the proximal diameter enlarged portion 26 of the needle insertion hole 41 in such a way that the position at which the melt-bond portion is to be formed does not reach the proximal diameter enlarged portion 26. In the case where the needle tube is not desired to bend, it is preferable to form the melt-bond portion at a position where the distal end of the melt-bond portion 45 is proximate to the distal diameter enlarged portion 44 of the needle insertion hole 41.

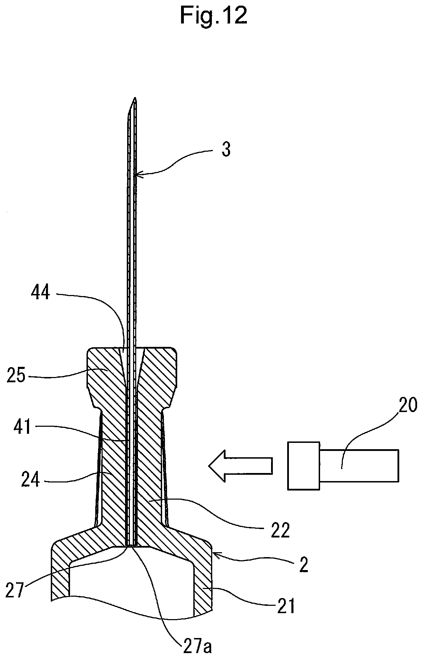

In the above-described embodiment, in the process of inserting the needle tube 3 into the needle insertion hole, the needle tube 3 is placed in position by using the needle supporting member 12 separate from the outer cylinder 2. But the needle tube 3 may be placed in position by providing the outer cylinder with a construction different from the above-described one. Like another embodiment shown in Fig. 12, the needle tube 3 may be placed in position by bringing the needle tube into contact with a needle stop portion 27 formed inside the distal part 22 of the outer cylinder 2. The needle stop portion 27 is formed of an annular rib projected inward from the inner surface of the proximal end of the needle insertion hole 41. A communication hole 27a positioned at a central portion of the needle stop portion 27 allows communication between the needle insertion hole 41 and the main body part 21. Therefore the lumen of the needle tube 3 placed in position with the needle tube in contact with the needle stop portion 27 communicates with the internal accommodation portion of the main body part 21 through the communication hole 27a. In this manner, the needle tube 3 is placed in position with respect to the outer cylinder 2.

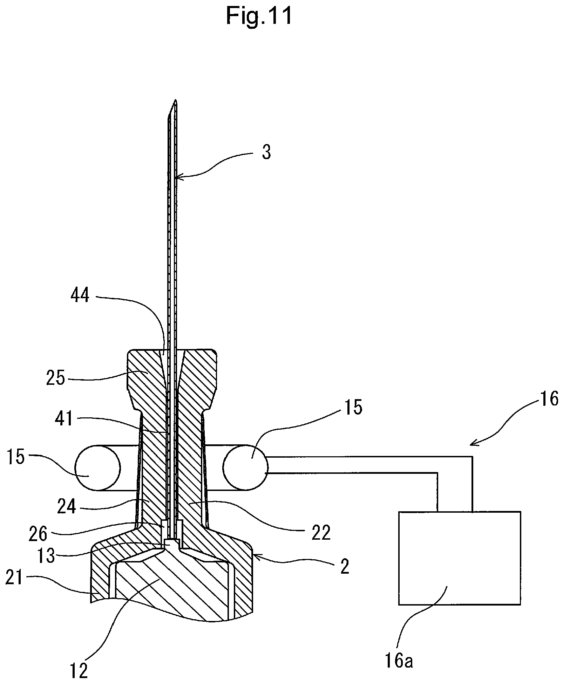

As an energy imparting means to be used in the melt-bond process of the production method of the present invention, it is preferable to use the above-described semiconductor laser irradiation apparatus. But as the energy imparting means, like an embodiment shown in Fig. 11, a high frequency induction heating apparatus 16 may be used. The high frequency induction heating apparatus 16 has a work coil 15 and a power supply 16a which applies an alternating current to the work coil 15. Upon application of the alternating current from the power supply 16a to the work coil 15, a magnetic field is generated on the periphery of the work coil 15 to generate an eddy current in the needle tube 3. Thereby the needle tube 3 generates heat.

### EXAMPLE

Outer cylinders (capacity: 1mL) having a configuration as shown in Figs. 5 and 6 were produced by using cyclic olefin polymer (COP produced by Nippon Zeon Co., Ltd.). The inner diameter of the needle insertion hole disposed at the distal part of the outer cylinder was 0.43mm. Needle tubes made of stainless steel of 27G (outer diameter: about 0.41mm) and 29G (outer diameter: about 0.34mm) were used. As shown in Figs. 9 and 10, to heat the needle tubes, a portion of each needle tube positioned at a side a little distal from the proximal portion thereof was irradiated by using the semiconductor laser irradiation apparatus in a state in which the outer cylinder was substantially vertically disposed and the needle supporting member 12 was disposed inside the outer cylinder. In the initial heating process (strong heating process), the output of the semiconductor laser irradiation apparatus, the laser focus diameter, and the laser irradiation period of time were set to 18W, ϕ3m, and 0.5 seconds respectively. After the execution of the initial heating process finished, the final heating process was performed in succession. The final heating process was performed in a manner similar to that of the initial heating process except that the output of the laser irradiation and the laser irradiation period of time were set to 7W and two seconds respectively. After the execution of the final heating process finished, the outer cylinders were naturally cooled. In this manner, a plurality of the needle-mounted outer cylinders of an embodiment of the present invention was produced.

### EXPERIMENT

As a result of visual observation of the needle-mounted outer cylinder (27G was used. Example 1) and the needle-mounted outer cylinder (29G was used. Example 2) produced in the above-described example, the generation of bubbles at the distal part thereof and distortion and deformation of the distal part thereof were not recognized. As a result of the measurement of the length of the melt-bond portion in the axial direction of the outer cylinder, the length of each needle-mounted outer cylinder was about 3mm.

The pull-out strength of the needle of the needle-mounted outer cylinder of each of the examples 1 and 2 (six pieces respectively) was measured in accordance with JIS 3209, 2011. The pull-out strength of the needle of the needle-mounted outer cylinder of the example 1 was 70 to 110N. The pull-out strength of the needle of the needle-mounted outer cylinder of the example 2 was 50 to 90N.

### INDUSTRIAL APPLICABILITY

The needle-mounted outer cylinder of the present invention is as described below.
(1) A needle-mounted outer cylinder comprises a metal needle tube having a lumen penetrating therethrough from a distal end thereof to a proximal end thereof and an outer cylinder, made of thermoplastic resin, which has a distal part having a needle insertion hole into which a proximal portion of said needle tube is inserted; and said proximal portion of said needle tube is bonded to said outer cylinder with a melt-bond portion formed inside said needle insertion hole by melting an inner surface of said needle insertion hole of said outer cylinder, wherein said needle insertion hole has an inner diameter a little larger than an outer diameter of said proximal portion of said needle tube; said melt-bond portion is positioned at a side distal by a predetermined length from a proximal end of said needle insertion hole and is formed by partly melting said inner surface of said needle insertion hole, by flowing down toward a proximal side of said needle insertion hole and by filling a gap between an outer surface of said proximal portion of said needle tube and said inner surface of said needle insertion hole with a portion of said inner surface of said needle insertion hole; and said distal part of said outer cylinder does not substantially have bubbles to be formed when said melt-bond portion is formed.
   In the needle-mounted outer cylinder, because the needle tube is securely fixed to the needle insertion hole formed at the distal part of the outer cylinder, the needle tube does not separate from the outer cylinder during use.
(2) A needle-mounted outer cylinder according to the above (1), wherein said melt-bond portion is formed by heating said thermoplastic resin in such a way that a temperature of said thermoplastic resin becomes not less than a melting temperature thereof and less than a thermal decomposition temperature thereof and thereafter solidifying said thermoplastic resin.
(3) A needle-mounted outer cylinder according to the above (1) or (2), wherein said needle insertion hole has a diameter enlarged portion formed of a melted surface extended from said distal end of said melt-bond portion.

The method of producing the needle-mounted outer cylinder of the present invention is as follows:
(4) A method of producing a needle-mounted outer cylinder comprises a metal needle tube having a lumen penetrating therethrough from a distal end thereof to a proximal end thereof and an outer cylinder, made of thermoplastic resin, which has a distal part having a needle insertion hole into which a proximal portion of said needle tube is inserted; and said proximal portion of said needle tube is bonded to said outer cylinder with a melt-bond portion formed inside said needle insertion hole by melting an inner surface of said needle insertion hole of said outer cylinder, said method of producing said needle-mounted outer cylinder consists of execution of a melt-bond process of melting and bonding said proximal portion of said needle tube to said outer cylinder in a state in which said needle tube is inserted into said needle insertion hole of said outer cylinder and in which said outer cylinder is erect; and said melt-bond process being performed by imparting energy to said needle tube at a portion thereof positioned at a side distal from a proximal end of said needle insertion hole and from said proximal end of said needle tube to allow said needle tube to generate heat so that resin positioned at a heat generation portion and forming said inner surface of said needle insertion hole is melted, and thereafter by flowing said melted resin downward inside said needle insertion hole to thereby fill a gap between an outer surface of said proximal portion of said needle tube and said needle insertion hole.
   According to the production method of the present invention, it is possible to easily and securely produce the needle-mounted outer cylinder which allows a needle tube insertion work to be performed easily by setting into the needle insertion hole formed at the distal part of the outer cylinder larger than the outer diameter of the needle tube and which hardly generates foaming at the melt-bond portion and foaming-caused defective fixing of the needle tube to the outer cylinder.
(5) A method of producing a needle-mounted outer cylinder according to the above (4), wherein said melt-bond process includes a needle tube heating process of allowing said needle tube to generate heat in such a way that a thermoplastic resin heating temperature at said heat generation portion becomes not less than a melting temperature of said thermoplastic resin and less than a thermal decomposition temperature thereof; and a solidifying process of solidifying said melted resin subsequently to finish of execution of said needle tube heating process.
(6) A method of producing a needle-mounted outer cylinder according to the above (4), wherein said melt-bond process consists of an initial heating process of allowing said needle tube to generate heat in such a way that a thermoplastic resin heating temperature at said heat generation portion exceeds a thermal decomposition temperature of said thermoplastic resin and execution of a final heating process of allowing said needle tube to generate heat in such a way that said thermoplastic resin heating temperature at said heat generation portion becomes not less than a melting temperature of said thermoplastic resin and less than said thermal decomposition temperature thereof, after execution of said initial heating process finishes.
(7) A method of producing a needle-mounted outer cylinder according to the above (6), wherein said needle tube is allowed to generate heat by irradiating said needle tube with laser or high-frequency induction heating; and said initial heating process is performed by irradiating said needle tube with laser or high-frequency induction heating at a first output level for a predetermined period of time; and said final heating process is performed by irradiating said needle tube with laser or high-frequency induction heating for a predetermined period of time at a level lower than said first output level.
(8) A method of producing a needle-mounted outer cylinder according to the above (7), wherein said final heating process is performed to allow said needle tube to generate heat at a second output level lower than said first output level.
(9) A method of producing a needle-mounted outer cylinder according to the above (7), wherein said final heating process is performed to allow said needle tube to generate heat by gradually lowering said first output level.
(10) A method of producing a needle-mounted outer cylinder according to any one of the above (4) through (9), wherein said melt-bond process is performed in such a way that an outer surface of said distal part of said outer cylinder does not reach a melting temperature of said resin and in a state in which a stress loading is not applied to said distal part of said outer cylinder from said outer surface thereof.

## Claims

1. A needle-mounted outer cylinder for a syringe comprising a metal needle tube having a lumen penetrating therethrough from a distal end thereof to a proximal end thereof and an outer cylinder, made of thermoplastic resin, which has a distal part having a needle insertion hole into which a proximal portion of said needle tube is inserted; and said proximal portion of said needle tube is bonded to said outer cylinder with a melt-bond portion formed inside said needle insertion hole by melting an inner surface of said needle insertion hole of said outer cylinder,
wherein said needle insertion hole has an inner diameter a little larger than an outer diameter of said proximal portion of said needle tube so as to form a gap; said melt-bond portion is positioned at a distal end side distal by a predetermined length from a proximal end of said needle insertion hole and is formed by partly melting of a part of said inner surface of said needle insertion hole immediately distal to said melt-bond portion, by flowing down toward a proximal end of said needle insertion hole and by filling the gap between the outer surface of said proximal portion of said needle tube and said inner surface of said needle insertion hole with a portion of said inner surface of said needle insertion hole thus forming the melt-bond portion; and said distal part of said outer cylinder does not substantially have bubbles to be formed when said melt-bond portion is formed.

2. A needle-mounted outer cylinder according to claim 1, wherein said melt-bond portion is formed by heating said thermoplastic resin in such a way that a temperature of said thermoplastic resin becomes not less than a melting temperature thereof and less than a thermal decomposition temperature thereof and thereafter solidifying said thermoplastic resin.

3. A needle-mounted outer cylinder according to claim 1 or 2, wherein said needle insertion hole has a diameter enlarged portion formed of a melted surface extended from said distal end of said melt-bond portion.

4. A method of producing a needle-mounted outer cylinder for a syringe comprising a metal needle tube having a lumen penetrating therethrough from a distal end thereof to a proximal end thereof and an outer cylinder, made of thermoplastic resin, which has a distal part having a needle insertion hole into which a proximal portion of said needle tube is inserted; and said proximal portion of said needle tube is bonded to said outer cylinder with a melt-bond portion formed inside said needle insertion hole by melting an inner surface of said needle insertion hole of said outer cylinder,
said method of producing said needle-mounted outer cylinder consists of execution of a melt-bond process of melting and bonding said proximal portion of said needle tube to said outer cylinder in a state in which said needle tube is inserted into said needle insertion hole of said outer cylinder and in which said outer cylinder is erect; and said melt-bond process being performed by imparting energy to said needle tube at a portion thereof positioned at a side distal from a proximal end of said needle insertion hole and from said proximal end of said needle tube to allow said needle tube to generate heat so that resin positioned at a heat generation portion and forming said inner surface of said needle insertion hole is melted, and thereafter by flowing said melted resin downward inside said needle insertion hole to thereby fill a gap between an outer surface of said proximal portion of said needle tube and said needle insertion hole thus forming the melt-bond portion.

5. A method of producing a needle-mounted outer cylinder according to claim 4, wherein said melt-bond process includes a needle tube heating process of allowing said needle tube to generate heat in such a way that a thermoplastic resin heating temperature at said heat generation portion becomes not less than a melting temperature of said thermoplastic resin and less than a thermal decomposition temperature thereof; and a solidifying process of solidifying said melted resin subsequently to finish of execution of said needle tube heating process.

6. A method of producing a needle-mounted outer cylinder according to claim 4, wherein said melt-bond process consists of an initial heating process of allowing said needle tube to generate heat in such a way that a thermoplastic resin heating temperature at said heat generation portion exceeds a thermal decomposition temperature of said thermoplastic resin and execution of a final heating process of allowing said needle tube to generate heat in such a way that said thermoplastic resin heating temperature at said heat generation portion becomes not less than a melting temperature of said thermoplastic resin and less than said thermal decomposition temperature thereof, after execution of said initial heating process finishes.

7. A method of producing a needle-mounted outer cylinder according to claim 6, wherein said needle tube is allowed to generate heat by irradiating said needle tube with laser or high-frequency induction heating; and said initial heating process is performed by irradiating said needle tube with laser or high-frequency induction heating at a first output level for a predetermined period of time; and said final heating process is performed by irradiating said needle tube with laser or high-frequency induction heating for a predetermined period of time at a level lower than said first output level.

8. A method of producing a needle-mounted outer cylinder according to claim 7, wherein said final heating process is performed to allow said needle tube to generate heat at a second output level lower than said first output level.

9. A method of producing a needle-mounted outer cylinder according to claim 7, wherein said final heating process is performed to allow said needle tube to generate heat by gradually lowering said first output level.

10. A method of producing a needle-mounted outer cylinder according to any one of claims 4 through 9, wherein said melt-bond process is performed in such a way that an outer surface of said distal part of said outer cylinder does not reach a melting temperature of said resin and in a state in which a stress loading is not applied to said distal part of said outer cylinder from said outer surface thereof.

## Patentansprüche

1. An einer Nadel montierter Außenzylinder für eine Spritze, die ein Nadelrohr aus Metall mit einem sich von einem distalen Ende desselben zu einem proximalen Ende desselben erstreckenden Lumen und einem aus thermoplastischem Harz bestehenden Außenzylinder umfasst, der einen distalen Teil mit einem Nadeleinsteckloch besitzt, in das ein proximaler Abschnitt des Nadelrohres eingesetzt ist; und der proximale Abschnitt des Nadelrohres ist mit dem Außenzylinder verklebt, wobei ein Schmelzklebeabschnitt in dem Nadeleinsteckloch durch Schmelzen einer Innenfläche des Nadeleinstecklochs des Außenzylinders gebildet wird,
wobei das Nadeleinsteckloch einen Innendurchmesser besitzt, der etwas größer ist als ein Außendurchmesser des proximalen Abschnitts des Nadelrohres, so dass ein Spalt entsteht;
wobei der Schmelzklebeabschnitt an einer distalen Stirnseite positioniert ist, die um eine vorbestimmte Länge von einem proximalen Ende des Nadeleinsteckloches beabstandet ist, und dadurch gebildet wird, dass ein Teil der Innenfläche des zu dem Schmelzklebeabschnitt unmittelbar distalen Nadeleinsteckloches teilweise geschmolzen wird, in Richtung zu einem proximalen Ende des Nadeleinsteckloches nach unten fließt und den Spalt zwischen der Außenfläche des proximalen Abschnitts des Nadelrohres und der Innenfläche des Nadeleinsteckloches füllt, wobei ein Abschnitt der Innenfläche des Nadeleinsteckloches so den Schmelzklebeabschnitt bildet; und
sich im distalen Teil des Außenzylinders im Wesentlichen keine Blasen bilden, wenn der Schmelzklebeabschnitt gebildet wird.

2. An einer Nadel montierter Außenzylinder nach Anspruch 1, wobei der Schmelzklebeabschnitt durch Erwärmen des thermoplastischen Harzes in einer solchen Weise gebildet wird, dass eine Temperatur des thermoplastischen Harzes nicht kleiner wird als eine Schmelztemperatur desselben und kleiner als eine thermische Zersetzungstemperatur desselben, und danach das thermoplastische Harz verfestigt wird.

3. An einer Nadel montierter Außenzylinder nach Anspruch 1 oder 2, wobei das Nadeleinsteckloch einen aus einer geschmolzenen Oberfläche gebildeten Abschnitt mit größerem Durchmesser besitzt, der sich von dem distalen Ende des Schmelzklebeabschnitts erstreckt.

4. Verfahren zur Herstellung eines an einer Nadel montierten Außenzylinders für eine Spritze, die ein Nadelrohr aus Metall mit einem sich von einem distalen Ende desselben zu einem proximalen Ende desselben erstreckenden Lumen und einem aus thermoplastischem Harz bestehenden Außenzylinder umfasst, der einen distalen Teil mit einem Nadeleinsteckloch besitzt, in das ein proximaler Abschnitt des Nadelrohres eingesetzt ist; und der proximale Abschnitt des Nadelrohres ist mit dem Außenzylinder verklebt, wobei ein Schmelzklebeabschnitt in dem Nadeleinsteckloch durch Schmelzen einer Innenfläche des Nadeleinsteckloches des Außenzylinders gebildet wird,
wobei das Verfahren zur Herstellung des an einer Nadel montierten Außenzylinders in der Ausführung eines Schmelzklebevorgangs besteht, bei dem der proximale Abschnitt des Nadelrohres in einem Zustand geschmolzen und mit dem Außenzylinder verklebt wird, in dem das Nadelrohr in das Nadeleinsteckloch des Außenzylinders eingesetzt ist und in dem der Außenzylinder aufrecht ist; und wobei das Schmelzklebeverfahren durchgeführt wird, indem das Nadelrohr an einem Abschnitt mit Energie beaufschlagt wird, der auf einer Seite positioniert ist, die distal von einem proximalen Ende des Nadeleinsteckloches und von dem proximalen Ende des Nadelrohres gelegen ist, damit das Nadelrohr Wärme erzeugen kann, damit an einem Wärmeerzeugungsabschnitt befindliches Harz, das die Innenfläche des Nadeleinsteckloches bildet, geschmolzen wird, und indem das geschmolzene Harz danach in dem Nadeleinsteckloch nach unten fließt, um dadurch einen Spalt zwischen einer Außenfläche des proximalen Abschnitts des Nadelrohres und dem Nadeleinsteckloch zu füllen, so dass der Schmelzklebeabschnitt entsteht.

5. Verfahren zur Herstellung eines an einer Nadel montierten Außenzylinders nach Anspruch 4, wobei der Schmelzklebevorgang einen Nadelrohrerwärmungsvorgang umfasst, bei dem man das Nadelrohr in einer solchen Weise Wärme erzeugen lässt, dass eine Erwärmungstemperatur des thermoplastischen Harzes an dem Wärmeerzeugungsabschnitt nicht kleiner wird als eine Schmelztemperatur des thermoplastischen Harzes und kleiner als eine thermische Zersetzungstemperatur desselben; und einen Verfestigungsvorgang, bei dem das geschmolzene Harz anschließend verfestigt wird, um die Ausführung des Nadelrohrerwärmungsvorgangs zu beenden.

6. Verfahren zur Herstellung eines an einer Nadel montierten Außenzylinders nach Anspruch 4, wobei der Schmelzklebevorgang aus einem anfänglichen Erwärmungsvorgang besteht, bei dem man das Nadelrohr in einer solchen Weise Wärme erzeugen lässt, dass eine Erwärmungstemperatur des thermoplastischen Harzes an dem Wärmeerzeugungsabschnitt größer ist als eine thermische Zersetzungstemperatur des thermoplastischen Harzes, und aus der Ausführung eines abschließenden Erwärmungsvorgangs, bei dem man das Nadelrohr in einer solchen Weise Wärme erzeugen lässt, dass die Erwärmungstemperatur des thermoplastischen Harzes an dem Wärmeerzeugungsabschnitt nicht kleiner wird als eine Schmelztemperatur des thermoplastischen Harzes und kleiner als dessen thermische Zersetzungstemperatur, nachdem die Ausführung des anfänglichen Erwärmungsvorgangs beendet ist.

7. Verfahren zur Herstellung eines an einer Nadel montierten Außenzylinders nach Anspruch 6, wobei man das Nadelrohr durch Bestrahlung des Nadelrohres mit Lasererwärmung oder hochfrequenter Induktionserwärmung Wärme erzeugen lässt; und der anfängliche Erwärmungsvorgang durch Bestrahlung des Nadelrohres mit Lasererwärmung oder hochfrequenter Induktionserwärmung auf einem ersten Ausgangspegel für eine vorbestimmte Zeitdauer durchgeführt wird; und der abschließende Erwärmungsvorgang durch Bestrahlung des Nadelrohres mit Lasererwärmung oder hochfrequenter Induktionserwärmung für eine vorbestimmte Zeitdauer bei einem niedrigeren Pegel als dem ersten Ausgangspegel durchgeführt wird.

8. Verfahren zur Herstellung eines an einer Nadel montierten Außenzylinders nach Anspruch 7, wobei der abschließende Erwärmungsvorgang durchgeführt wird, um das Nadelrohr auf einem zweiten Ausgangspegel, der niedriger ist als der erste Ausgangspegel, Wärme erzeugen zu lassen.

9. Verfahren zur Herstellung eines an einer Nadel montierten Außenzylinders nach Anspruch 7, wobei der abschließende Erwärmungsvorgang durchgeführt wird, um das Nadelrohr unter allmählicher Absenkung des ersten Ausgangspegels Wärme erzeugen zu lassen.

10. Verfahren zur Herstellung eines an einer Nadel montierten Außenzylinders nach einem der Ansprüche 4 bis 9, wobei der Schmelzklebevorgang auf eine solche Weise durchgeführt wird, das eine Außenfläche des distalen Teils des Außenzylinders eine Schmelztemperatur des Harzes nicht erreicht, und in einem Zustand, in dem keine Spannungsbelastung von der Außenfläche des Außenzylinders auf dessen distalen Teil aufgebracht wird.

## Revendications

1. Cylindre extérieur monté sur une aiguille destiné à une seringue, comprenant un tube à aiguille en métal comportant une lumière le traversant de son extrémité distale à son extrémité proximale et un cylindre extérieur, en résine thermoplastique, qui comprend une partie distale ayant un trou d'insertion d'aiguille dans lequel une partie proximale dudit tube à aiguille est insérée ; et ladite partie proximale dudit tube à aiguille est liée audit cylindre extérieur par une partie de liaison par fusion formée à l'intérieur dudit trou d'insertion d'aiguille en faisant fondre une surface intérieure dudit trou d'insertion d'aiguille dudit cylindre extérieur,
dans lequel ledit trou d'insertion d'aiguille a un diamètre intérieur un peu plus grand qu'un diamètre extérieur de ladite partie proximale dudit tube à aiguille de manière à former un espace ; ladite partie de liaison par fusion est placée au niveau d'un côté d'extrémité distale, distal d'une longueur prédéterminée par rapport à une extrémité proximale dudit trou d'insertion d'aiguille, et est formée par la fusion partielle d'une partie de ladite surface intérieure dudit trou d'insertion d'aiguille immédiatement distale par rapport à ladite partie de liaison par fusion, en s'écoulant vers le bas vers une extrémité proximale dudit trou d'insertion d'aiguille et en remplissant l'espace entre la surface extérieure de ladite partie proximale dudit tube à aiguille et ladite surface intérieure dudit trou d'insertion d'aiguille avec une partie de ladite surface intérieure dudit trou d'insertion d'aiguille en formant ainsi la partie de liaison par fusion ; et ladite partie distale dudit cylindre extérieur ne présente sensiblement pas de bulles formées lorsque la partie de liaison par fusion est formée.

2. Cylindre extérieur monté sur une aiguille selon la revendication 1, dans lequel ladite partie de liaison par fusion est formée en chauffant ladite résine thermoplastique de telle sorte que la température de ladite résine thermoplastique n'est pas inférieure à sa température de fusion et n'est pas inférieure à sa température de désintégration thermique, et en solidifiant ensuite ladite résine thermoplastique.

3. Cylindre extérieur monté sur une aiguille selon la revendication 1 ou 2, dans lequel ledit trou d'insertion d'aiguille a une partie de diamètre agrandi formée d'une surface fondue qui s'étend à partir de ladite extrémité distale de ladite partie de liaison par fusion.

4. Procédé de fabrication d'un cylindre extérieur monté sur une aiguille destiné à une seringue,
comprenant un tube à aiguille en métal comportant une lumière le traversant de son extrémité distale à son extrémité proximale et un cylindre extérieur, en résine thermoplastique, qui comprend une partie distale ayant un trou d'insertion d'aiguille dans lequel une partie proximale dudit tube à aiguille est insérée ; et ladite partie proximale dudit tube à aiguille est liée audit cylindre extérieur par une partie de liaison par fusion formée à l'intérieur dudit trou d'insertion d'aiguille en faisant fondre une surface intérieure dudit trou d'insertion d'aiguille dudit cylindre extérieur,
ledit procédé de fabrication dudit cylindre extérieur monté sur une aiguille comprend l'exécution d'une opération de liaison par fusion consistant à faire fondre et à lier ladite partie proximale dudit tube à aiguille audit cylindre extérieur dans un état où ledit tube à aiguille est inséré dans ledit trou d'insertion d'aiguille dudit tube à aiguille et où ledit cylindre extérieur est dressé ; et ladite opération de liaison par fusion est effectuée en appliquant de l'énergie audit tube à aiguille dans une partie de celui-ci positionnée au niveau d'un côté distal par rapport à une extrémité proximale dudit trou d'insertion d'aiguille et par rapport à ladite extrémité proximale dudit tube à aiguille de manière à permettre audit tube à aiguille de générer de la chaleur de telle sorte que la résine placée au niveau d'une partie de production de chaleur et formant ladite surface intérieure dudit trou d'insertion d'aiguille est fondue, et en faisant ensuite s'écouler ladite résine fondue vers le bas à l'intérieur dudit trou d'insertion d'aiguille pour ainsi remplir un espace entre une surface extérieure de ladite partie proximale dudit tube à aiguille et ledit trou d'insertion d'aiguille en formant ainsi la partie de liaison par fusion.

5. Procédé de fabrication d'un cylindre extérieur monté sur une aiguille selon la revendication 4, dans lequel ladite opération de liaison par fusion comprend une opération de chauffage de tube à aiguille consistant à permettre audit tube à aiguille de produire de la chaleur de telle manière qu'une température de chauffage de la résine thermoplastique au niveau de ladite partie de production de chaleur n'est pas inférieure à une température de fusion de ladite résine thermoplastique et n'est pas inférieure à sa température de désintégration ; et une opération de solidification consistant à solidifier ensuite ladite résine fondue pour terminer l'exécution de ladite opération de chauffage du tube à aiguille.

6. Procédé de fabrication d'un cylindre extérieur monté sur une aiguille selon la revendication 4, dans lequel ladite opération de liaison par fusion comprend une opération de chauffage initial consistant à permettre audit tube à aiguille de produire de la chaleur de telle manière qu'une température de chauffage de la résine thermoplastique au niveau de ladite partie de production de chaleur dépasse une température de désintégration thermique de ladite résine thermoplastique et l'exécution d'une opération de chauffage final consistant à permettre audit tube à aiguille de produire de la chaleur de telle manière que ladite température de chauffage de la résine thermoplastique au niveau de ladite partie de production de chaleur n'est pas inférieure à une température de fusion de ladite résine thermoplastique et n'est pas inférieure à ladite température de désintégration thermique de celle-ci, une fois que l'exécution de ladite opération de chauffage initial est terminée.

7. Procédé de fabrication d'un cylindre extérieur monté sur une aiguille selon la revendication 6, dans lequel ledit tube à aiguille peut générer de la chaleur en irradiant ledit tube à aiguille avec un chauffage par laser ou un chauffage par induction haute fréquence ; et ladite opération de chauffage initial est effectuée en irradiant ledit tube à aiguille avec un chauffage par laser ou un chauffage par induction haute fréquence à un premier niveau de sortie pendant une période de temps prédéterminée ; et ladite opération de chauffage final est effectuée en irradiant ledit tube à aiguille avec un chauffage par laser ou un chauffage par induction haute fréquence pendant une période de temps prédéterminée à un niveau inférieur audit premier niveau de sortie.

8. Procédé de fabrication d'un cylindre extérieur monté sur une aiguille selon la revendication 7, dans lequel ladite opération de chauffage final est effectuée de manière à permettre audit tube à aiguille de produire de la chaleur à un second niveau de sortie inférieur audit premier niveau de sortie.

9. Procédé de fabrication d'un cylindre extérieur monté sur une aiguille selon la revendication 7, dans lequel ladite opération de chauffage final est effectuée de manière à permettre audit tube à aiguille de produire de la chaleur en abaissant progressivement ledit premier niveau de sortie.

10. Procédé de fabrication d'un cylindre extérieur monté sur une aiguille selon l'une quelconque des revendications 4 à 9, dans lequel ladite opération de liaison par fusion est effectuée de telle sorte qu'une surface extérieure de ladite partie distale dudit cylindre extérieur n'atteint pas une température de fusion de ladite résine et dans un état où une charge de contrainte n'est pas appliquée à ladite partie distale dudit cylindre extérieur depuis ladite surface extérieure de celui-ci.
